# EUROPÄISCHE PATENTSCHRIFT

(11) **EP 1 165 037 B1**
(45) Veröffentlichungstag und Bekanntmachung des Hinweises auf die Patenterteilung: **12.01.2005**
(21) Anmeldenummer: 00916895.6
(22) Anmeldetag: 03.03.2000
(51) Int. Cl.: A61K 7/48, A61K 7/42

(54) **VERWENDUNG VON NANOSKALIGEN WASSERLÖSLICHEN $g(b)-(1,3)-GLUCANEN**
USE OF NANOSCALAR WATER-SOLUBLE $g(b)-(1,3) GLUCANS
UTILISATION DE $g(b)-(1,3)-GLUCANES NANOMETRIQUES HYDROSOLUBLES

(30) Priorität: 12.03.1999 DE 19911058
(43) Veröffentlichungstag der Anmeldung: 02.01.2002
(73) Patentinhaber: Biotec Pharmacon ASA, 9008 Tromsö (NO)
(72) Erfinder: KROPF, Christian, D-40597 Düsseldorf (DE); GRIESBACH, Ute, D-40597 Düsseldorf (DE); FABRY, Bernd, D-41352 Korschenbroich (DE); ENGSTAD, Rolf, E., N-9008 Tromso (NO)
(74) Vertreter: Melzer, Wolfgang, Dipl.-Ing.
(86) Internationale Anmeldenummer: PCT/EP2000/001829
(87) Internationale Veröffentlichungsnummer: WO 2000/054741

(56) Entgegenhaltungen:
- WO-A-95/30022
- WO-A-99/11695
- US-A- 5 705 184
- DATABASE WPI Section Ch, Week 198015 Derwent Publications Ltd., London, GB; Class D21, AN 1980-25985C XP002141792 & JP 55 027126 A (ASAHI CHEM IND CO LTD), 27. Februar 1980 (1980-02-27)

## Beschreibung

### Gebiet der Erfindung

Die Erfindung befindet sich auf dem Gebiet der Nanopartikel und betrifft die Verwendung von speziellen nanoskaligen β-(1,3)-Glucanen in der Kosmetik.

### Stand der Technik

Unter der Bezeichnung Glucane werden Homopolysaccharide auf Basis der Glucose verstanden. Je nach sterischer Verknüpfung unterscheidet man zwischen β-(1,3)-, β-(1,4)- und β-(1,6)-Glucanen. β-(1,3)-Glucane weisen meist eine helicale Struktur auf, während Glucane mit einer 1,4-Verknüpfung im allgemeinen eine lineare Struktur besitzen. Glucane und deren Derivate sind verschiedentlich für den Einsatz in der Kosmetik vorgeschlagen worden. So ist beispielsweise aus der Patentschrift **US 5,223,491** ein carboxymethyliertes β-1,3-Glucan für die topische Anwendung bekannt, das aus dem Hefepilz *Saccharomyces cerevisiae* extrahiert wurde. Das Glucan ist jedoch wasserunlöslich und kann daher nur mit großen Schwierigkeiten formuliert werden. Aus der Europäischen Patentschrift **EP-B1 0500718** (Donzis) ist der Einsatz von wasserunlöslichen β-(1,3)-Glucanen, die aus den Zellwänden von Hefen gewonnen werden, zur Revitalisierung der Haut bekannt. Diese Glucane sind jedoch wasserunlöslich und lassen sich daher ebenfalls nur schwer in kosmetische Zubereitungen einarbeiten. Gegenstand der internationalen Patentanmeldung **WO 98/40082** (Henkel) ist zwar die Verwendung von wasserlöslichen β-(1,3)-Glucanen als Wirkstoffe für die Hautbehandlung, die Glucane, bei denen es sich vorzugsweise um Schizopyhallan oder Krestin, also Pilzextrakte handelt, haben sich in der Praxis jedoch als nicht ausreichend wirksam erwiesen.

Die Wirkung der Glucane hängt stets mit der Geschwindigkeit zusammen, mit der die Verbindungen eingebaut bzw. resorbiert werden. Hier besteht für die verfügbaren Stoffe des Stands der Technik noch ein erhebliches Verbesserungspotential. Die Aufgabe der vorliegenden Erfindung hat somit darin bestanden, die Aufnahme von Glucanen bei topischer Applikation durch Bereitstellung neuer Anbietungsformen zu beschleunigen.

### Beschreibung der Erfindung

Gegenstand der Erfindung ist die Verwendung von nanoskaligen wasserlöslichen β-(1,3)-Glucanen, die im wesentlichen frei von (1,6)-Verknüpfungen sind und Teilchendurchmesser im Bereich von 10 bis 300 nm aufweisen, zur Herstellung von kosmetischen und/oder pharmazeutischen Zubereitungen.

Überraschenderweise wurde gefunden, daß sich die Resorption von wasserlöslichen β-(1,3)-Glucanen, die im wesentlichen frei von (1,6)-Verknüpfungen sind, sowohl durch das Stratum Comeum der Haut als auch die Keratinfibrillen des Haares signifikant steigern läßt, wenn diese in Form von Nanoteilchen, d.h. Partikeln mit einem mittleren Durchmesser im Bereich von 10 bis 300 und vorzugsweise 50 bis 150 nm vorliegen.

### Wasserlösliche β-(1,3)-Glucane

Die β-Glucane der Erfindung besitzen eine (1,3)-Struktur, d.h. sie sind weitgehend frei von unerwünschten (1,6)-Verknüpfungen. Vorzugsweise enthalten die Mittel Glucane, die auf Basis von Hefen der Familie *Saccharomyces,* speziell *Saccharomyces cerevisiae* erhalten werden. Glucane dieser Art sind in technischem Maße nach den Verfahren des Stands der Technik zugänglich. So beschreibt die internationale Patentanmeldung **WO 95130022** (Biotec-Mackzymal) ein Verfahre zur Herstellung solcher Stoffe, bei dem man Glucane mit β-(1,3)- und β-(1,6)-Verknüpfungen in solcher Weise mit β-(1,6)-Glucanasen in Kontakt bringt, daß praktisch alle β-(1,6)-Verknüpfungen gelöst werden. Vorzugsweise werden solche β -(1,3)-Glucane eingesetzt, deren Seitenketten ausschließlich (1,3)-Verknüpfungen aufweisen. Insbesondere werden zur Herstellung der Glucane Glucanasen auf Basis von *Trichodermia harzianum* eingesetzt. Soweit es die Herstellung und Zugänglichkeit der in den erfindungsgemäßen Mitteln enthaltenen Glucane angeht, wird in vollem Umfang auf die Offenbarung der oben genannten Schrift Bezug genommen.

### Herstellung von Nanopartikeln

Ein Verfahren zur Herstellung von Nanoteilchen durch rasche Entspannung von überkritischen Lösungen **(Rapid Expansion of Supercritical Solutions RESS)** ist beispielsweise aus dem Aufsatz von S.Chihlar, M.Türk und K.Schaber in **Proceedings World Congress on Particle Technology 3**, **Brighton, 1998** bekannt. Ein Weg zur Herstellung der Nanopartikel besteht darin, daß man
(a) die wasserlöslichen β-(1,3)-Glucane unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

Um zu verhindern, daß die Nanoteilchen wieder zusammenbacken, empfiehlt es sich, die Ausgangsstoffe in Gegenwart geeigneter Schutzkolloide oder Emulgatoren zu lösen und/oder die kritischen Lösungen in wäßrige und/oder alkoholische Lösungen der Schutzkolloide bzw. Emulgatoren oder aber in kosmetische Öle zu entspannen, welche ihrerseits wieder gelöste Emulgatoren und/oder Schutzkolloide enthalten können. Geeignete Schutzkolloide sind dabei z.B. Gelatine, Casein, Gummi arabicum, Lysalbinsäure, Stärke sowie Polymere, wie etwa Polyvinylalkohole, Polyvinylpyrrolidone Polyalkylenglycole und Polyacrylate. Die bevorzugt zu verwendenden nanoskaligen Glucane sind also die, die von einem Schutzkolloid und/oder einem Emulgator ummantelt vorliegen. Üblicherweise werden die Schutzkolloide oder Emulgatoren in Mengen von 0,1 bis 20, vorzugsweise 5 bis 15 Gew.-% - bezogen auf die Glucane - eingesetzt.

Ein weiteres geeignetes Verfahren zur Herstellung der nanoskaligen Teilchen bietet die **Evaporationstechnik**. Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, gegebenenfalls in Gegenwart einer darin gelösten oberflächenaktiven Verbindung gegeben, daß es durch die Homogenisierung der beiden nicht miteinander mischbaren Lösungsmittel zu einer Ausfällung der Nanoteilchen kommt, wobei das organische Lösungsmittel vorzugsweise verdampft. Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanoteilchen besteht in dem sogenannten **GAS-Verfahren** (Gas Anti Solvent Recrystallization). Das Verfahren nutzt ein hochkomprimiertes Gas oder überkritisches Fluid (z.B. Kohlendioxid) als Nicht-lösungsmittel zur Kristallisation von gelösten Stoffen. Die verdichtete Gasphase wird in die Primäriösung der Ausgangsstoffe eingeleitet und dort absorbiert, wodurch sich das Flüssigkeitsvolumen vergrößert, die Löslichkeit abnimmt und feinteilige Partikel ausgeschieden werden. Ähnlich geeignet ist das **PCA-Verfahren** (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim **PGSS-Verfahren** (Particles from Gas Saturated Solutions) werden die Ausgangsstoffe durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen.

### Gewerbliche Anwendbarkeit

Gegenüber Glucanen, speziell wasserlöslichen β-(1,3)-Glucanen, die ebenfalls weitgehend frei von unerwünschten (1,6)-Verknüpfungen sind, und damit den nächstliegenden Stand der Technik darstellen, bewirkt die besondere Feinteiligkeit der Partikel bei topischer Anwendung ihr rascheres Eindringen in das Stratum Comeum. Die Einsatzmenge der nanoskaligen Verbindungen liegt üblicherweise in der Größenordnung von 0,1 bis 5, vorzugsweise 0,5 bis 3 und insbesondere 1 bis 2 Gew.-% - bezogen auf die Zubereitungen.

### Kosmetische und/oder pharmazeutische Zubereitungen

Die unter erfindungsgemäßer Verwendung der nanoskaligen β-(1,3)-Glucane erhältlichen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparate, Puder oder Salben, können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristyterucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearytoleat, Stearylbehenat, Stearylerucat, Isostearylmyristat, Isostearylpalmitat, Isostearylstearat, Isostearylisostearat, Isostearyloleat, Isostearylbehenat, lsostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol. Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat, Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte VinylpyrrolidonNinylimidazol-Polymere, wie z.B. Luviquat® (BASF), Kondensationsprodukte von Polyglycolen und Aminen, quatemierte Kollagenpoiypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise-Vinylacetat/Crotonsäure-Copolymere, Vinylpytrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc. Cosm.Chem. 24, 281 (1973)**]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)**]. Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin-und Sitosterinsulfat bzw-phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyfester,
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metal-Ioxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfitter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Seien und dessen Derivate (z.B. Selen-Methionin), Slifbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können femer **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättem (Geranium, Patchouli, Petitgrain), Früchten (Anis, Koriander, Kümmel, Wacholder), Fruchtschalen (Bergamotte, Zitrone. Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linatylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citrat, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allyfamylgiycolat, Cyctavertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, lrotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Famesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Zur Herstellung der nanoskaligen Glucane (**Beispiele 1 bis 3**) wurde zunächst Kohlendioxid einem Reservoir mit einem konstanten Druck von 60 bar entnommen und über eine Kolonne mit einer Aktivkohle- und einer Molekularsieb-Packung gereinigt. Nach der Verflüssigung wurde das CO₂ mit Hilfe einer Diaphragma-Pumpe bei einer konstanten Fördermenge von 3,5 l/h auf den gewünschten überkritischen Druck p verdichtet. Anschließend wurde das Lösungsmittel in einem Vorheizer auf die erforderliche Temperatur T1 gebracht und in eine Extrakfionskolonne (Stahl, 400 ml) geleitet, welche mit dem Glucan beladen war. Die resultierende überkritische, d.h. fluide Mischung wurde über eine lasergezogene Düse (Länge 830 µm, Durchmesser 45 µm) bei einer Temperatur T2 in eine Plexiglas Expansionskammer versprüht, die eine 4 Gew.-%ige wäßrige Lösung eines Emulgators bzw. Schutzkolloids enthielt. Das fluide Medium verdampfte und zurück blieben die im Schutzkolloid eingeschlossenen, dispergierten Nanopartikel. Zur Herstellung der Nanoteilchen gemäß **Beispiel 4** wurde eine 1 Gew.-%ige wäßrige Glucanlösung unter starkem Rühren bei 40°C und einem verminderten Druck von 40 mbar in eine 4 Gew.-% wäßrige Lösung von Coco Glucosides getropft. Das verdampfende Lösungsmittel wurde in einer Kühlfalle kondensiert, während die Dispersion mit den Nanopartikeln zurückblieb. Die Verfahrensbedingungen und der mittlere Partikelgrößenbereich (photometrisch nach der 3-WEM-Methode bestimmt) sind in der nachfolgenden Tabelle 1 angegeben.

**Tabelle 1**

| **Nanopartikel** | | | | | | | |
|---|---|---|---|---|---|---|---|
| **Bsp.** | **Glucan** | **Lösungsmittel** | **p bar** | **T1 °C** | **T2 °C** | **Emulgator/Schutzkolloid** | **PGB nm** |
| 1 | Betaglucan* | CO₂ | 200 | 80 | 175 | Polyvinylalkohol | 50-125 |
| 2 | Betaglucan* | CO₂ | 180 | 70 | 160 | Polyethylenglycol (M=400) | 70-130 |
| 3 | Betaglucan* | CO₂ | 200 | 85 | 175 | Coco Glucosides | 50-150 |
| 4 | Betaglucan* | - | - | - | - | Coco Glucosides | 65-140 |

| | | | | | | | |
|---|---|---|---|---|---|---|---|
| *) Highcareen®GS Henkel KGaA / Düsseldorf | | | | | | | |

Die nachfolgende Tabelle 2 enthält eine Reihe von Formulierungsbeispielen mit Glucan-Nanopartikeln.

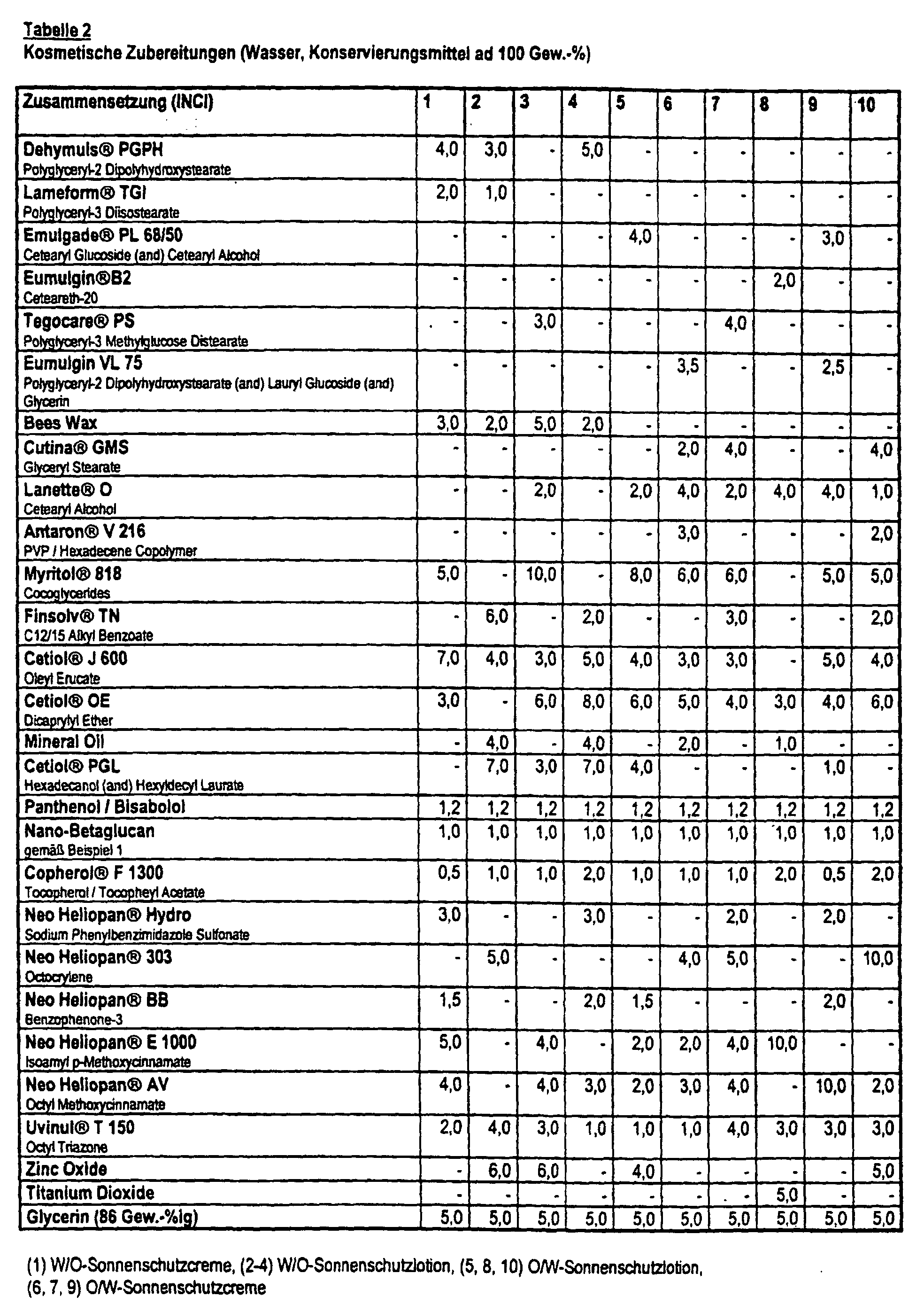

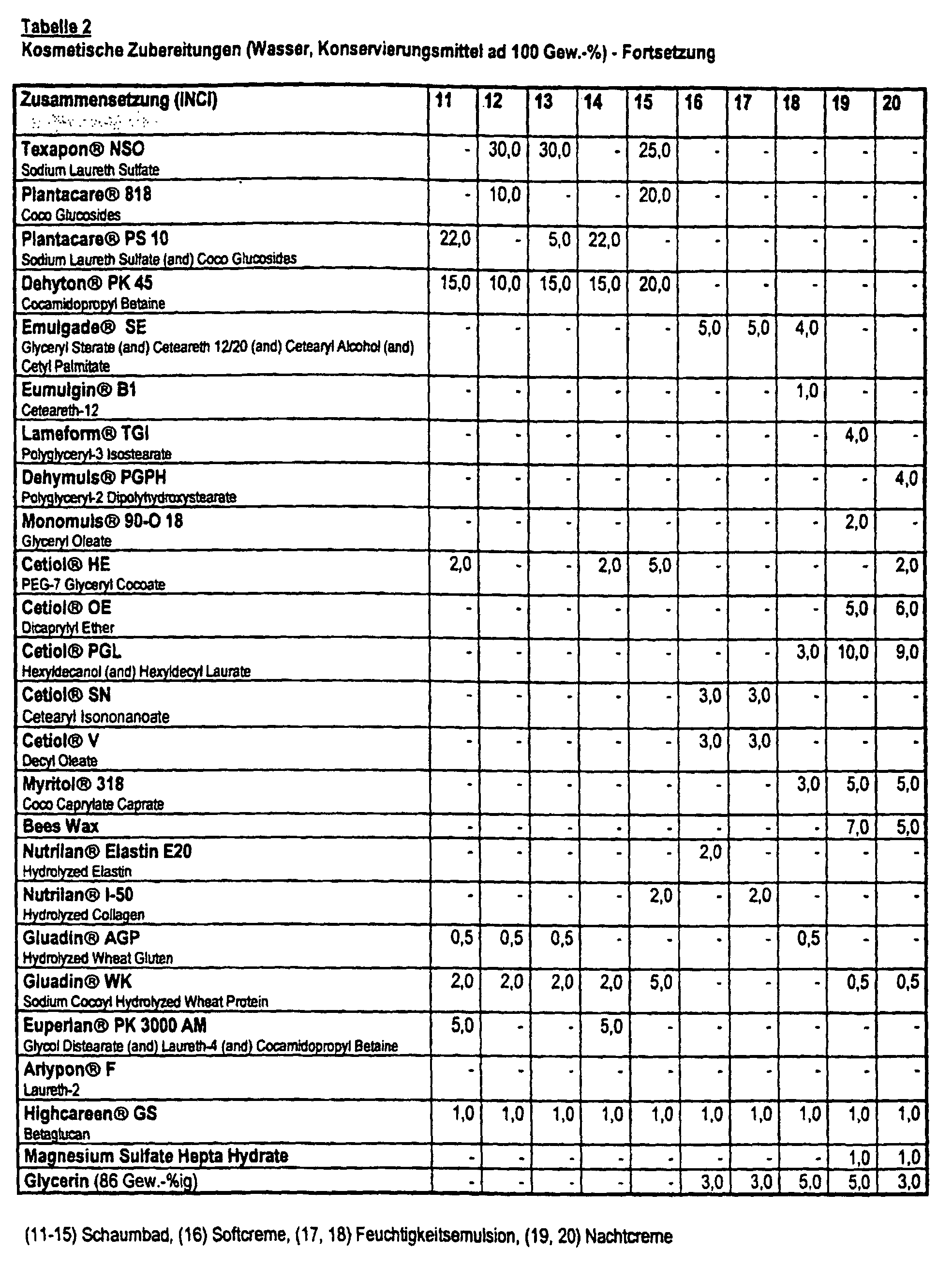

## Patentansprüche

1. Verwendung von nanoskaligen wasserlöslichen β-(1,3)-Glucanen, die im wesentlichen frei von β-(1,6)-Verknüpfungen.sind und Teilchendurchmesser im Bereich von 10 bis 300 nm aufweisen, zur Herstellung von kosmetischen und/oder pharmazeutischen Zubereitungen.

2. Verwendung nach Anspruch 1, **dadurch gekennzeichnet, daß** man Glucane einsetzt, die auf Basis von Hefen der Familie *Saccharomyces* erhalten werden.

3. Verwendung nach den Ansprüchen 1 und/oder 2, **dadurch gekennzeichnet, daß** man Glucane einsetzt, die erhalten werden, indem man Glucane mit β-(1,3)- und β-(1,6)-Verknüpfungen in solcher Weise mit β-(1,6)-Glucanasen in Kontakt bringt, daß praktisch alle β-(1,6-Verknüpfungen gelöst werden, und die Lyseprodukte dann in die nanoskalige Form bringt.

4. Verwendung nach Anspruch 3, **dadurch gekennzeichnet, daß** man Glucane einsetzt, die mit Glucanasen auf Basis von *Trichodermia harzianum* behandelt worden sind.

5. Verwendung nach mindestens einem der Ansprüche 1 bis 4, **dadurch gekennzeichnet, daß** man Nanopartikel einsetzt, welche von einem Schutzkolloid ummantelt vorliegen.

6. Verwendung nach Anspruch 5, **dadurch gekennzeichnet, daß** man als Schutzkolloid Polyvinylalkohol oder Polyethylenglycol einsetzt.

7. Verwendung nach mindestens einem der Ansprüche 1 bis 6, **dadurch gekennzeichnet, daß** man die Glucane in Mengen von 0,1 bis 5 Gew.-% - bezogen auf die Zubereitungen - einsetzt.

8. Verwendung nach mindestens einem der Ansprüche 1 bis 7, **dadurch gekennzeichnet, daß** man die Glucane zur Herstellung von Haarpflegemitteln einsetzt.

9. Verwendung nach mindestens einem der Ansprüche 1 bis 7, **dadurch gekennzeichnet, daß** man die Glucane zur Herstellung von Hautpflege- und Sonnenschutzmitteln einsetzt.

## Claims

1. Use of nanoscale water-soluble β-(1,3)-glucans which are essentially free from β - (1,6)-linkages and have particle diameters in the range from 10 to 300 nm for the preparation of cosmetic and/or pharmaceutical preparations.

2. Use according to Claim 1, **characterized in that** glucans are used which are obtained on the basis of yeasts of the *Saccharomyces* family.

3. Use according to Claims 1 and/or 2, **characterized in that** glucans are used which are obtained by bringing glucans with β-(1,3)- and β-(1,6)-linkages into contact with β-(1,6)-glucanases in such a way that virtually all of the β-(1,6)-linkages are broken, and the lysis products are then converted to nanoscale form.

4. Use according to Claim 3, **characterized in that** glucans are used which have been treated with glucanases based on *Trichodermia harzianum.*

5. Use according to at least one of Claims 1 to 4, **characterized in that** nanoparticles are used which are present in a form coated by a protective colloid.

6. Use according to Claim 5, **characterized in that** the protective colloid used is polyvinyl alcohol or polyethylene glycol.

7. Use according to at least one of Claims 1 to 6, **characterized in that** the glucans are used in amounts of from 0.1 to 5% by weight - based on the preparations.

8. Use according to at least one of Claims 1 to 7, **characterized in that** the glucans are used for the preparation of hair care compositions.

9. Use according to at least one of Claims 1 to 7, **characterized in that** the glucans are used for the preparation of skin care and sunscreen compositions.

## Revendications

1. Utilisation de β-(1,3)-glucanes nanométriques solubles dans l'eau, qui ne contiennent essentiellement pas de liaisons β-(1,6) et qui ont un diamètre de particule moyen situé dans un domaine de 10 à 300 nm, pour la préparation de compositions cosmétiques et/ou pharmaceutiques.

2. Utilisation selon la revendication 1, **caractérisée en ce que** l'on utilise des glucanes qui sont obtenus à partir de levures de la famille des *Saccharomyces*.

3. Utilisation selon les revendications 1 et/ou 2, **caractérisée en ce que** l'on utilise des glucanes que l'on a obtenus en mettant des glucanes ayant des Maisons β-(1,3) et β-(1,6) en contact avec des β-(1,6)-glucanases de manière que pratiquement toutes les liaisons β-(1,6) soient rompues, et en mettant ensuite les produits de lyse sous une forme nanométrique.

4. Utilisation selon la revendication 3, **caractérisée en ce que** l'on utilise des glucanes qui ont été traités avec des glucanases provenant de *Trichodermia harzianum.*

5. Utilisation selon au moins l'une des revendications 1 à 4, **caractérisée en ce que** l'on utilise des nanoparticules se présentant sous une forme enrobée par un colloïde protecteur.

6. Utilisation selon la revendication 5, **caractérisée en ce que** l'on utilise du poly(alcool vinylique) ou du polyéthylèneglycol comme colloïde protecteur.

7. Utilisation selon au moins l'une des revendications 1 à 6, **caractérisée en ce que** l'on utilise les glucanes en des quantités de 0,1 à 5 % en masse par rapport à la composition.

8. Utilisation selon au moins l'une des revendications 1 à 7, **caractérisée en ce que** l'on utilisé les glucanes pour la préparation de produits de soins capillaires.

9. Utilisation selon au moins l'une des revendications 1 à 7, **caractérisée en ce que** l'on utilise les glucanes pour la préparation de produits de soins pour la peau et de produits solaires.
